# EUROPEAN PATENT APPLICATION

(11) **EP 4 166 162 A1**
(43) Date of publication of application: **19.04.2023**
(21) Application number: 21821658.8
(22) Date of filing: 11.06.2021
(51) Int. Cl.: A61K 47/64, A61K 31/575, A61P 3/04, C07K 7/06, A61K 8/63, A61K 8/64, A61Q 19/06

(54) **DEOXYCHOLIC ACID-PEPTIDE CONJUGATE HAVING ANTI-OBESITY ACTIVITY AND USE THEREOF**

(30) Priority: 12.06.2020 KR 20200071657
(71) Applicant: Caregen Co., Ltd., Anyang-si, Gyeonggi-do 14119 (KR)
(72) Inventor: CHUNG, Yong Ji, Anyang-si, Gyeonggi-do 14119 (KR); KIM, Eun Mi, Anyang-si, Gyeonggi-do 14119 (KR); KIM, Seon Soo, Anyang-si, Gyeonggi-do 14119 (KR)
(74) Representative: Regimbeau
(86) International application number: PCT/KR2021/007325
(87) International publication number: WO 2021/251790

(57) **Abstract**

The present invention relates to: a deoxycholic acid-peptide conjugate having anti-obesity activity by inhibiting lipid production and promoting lipolysis; and a use for preventing, ameliorating or treating obesity comprising the conjugate as an active ingredient.

## Description

### [Technical Field]

The present invention relates to a deoxycholic acid-peptide conjugate having a structure in which deoxycholic acid and a peptide are chemically conjugated, and a use thereof.

### [Background Art]

Obesity refers to a state in which adipose tissue is excessively present in the body by accumulating excess energy as body fat when the balance with energy consumption consumed through food is not made. The obesity is recognized as an independent disease as well as a risk factor for various diseases such as cardiovascular disease and diabetes, but partial obesity, in which excess fat is deposited in a specific area such as the abdomen, is also closely related to these diseases. Accordingly, the partial obesity has a direct impact on health and quality of life, and also causes psychological atrophy and social maladaptation of individuals in terms of aesthetics.

The treatment of such partial obesity may be largely divided into surgical treatment and non-surgical treatment, and the surgical surgery may have side effects such as uneven skin surface and side effects of anesthesia. The non-surgical treatment has been variously used with lipolysis needles, ultrasound, high-frequency treatment, drug injection methods such as Meso therapy, a massage method, a gas injection method, and the like.

Meanwhile, deoxycholic acid as one of secondary bile acids as a metabolic by-product of intestinal bacteria was confirmed to induce an inflammatory action in adipose tissue to kill adipocytes (Rotunda et al., Dermatol Surg 30(7):1001-8(2004)), and was approved by the FDA and has been used as an ingredient in lipolytic injections. However, the deoxycholic acid may be accompanied by erythema, edema, bruising, hematoma, and pain at an administered site as a side effect when administered locally, and due to low solubility in water, an organic solvent is added to solubilize the deoxycholic acid, but there is a limit to cause side effects such as dermatitis and dry skin.

Under this background, the present inventors have conducted studies to develop a method for increasing anti-obesity activity while minimizing side effects caused by deoxycholic acid, and found that a deoxycholic acid-peptide conjugate in which a novel peptide synthesized by the present inventors and deoxycholic acid were chemically conjugated had high solubility in water and anti-obesity activity significantly improved compared to the use of a single substance, and then completed the present invention.

### [Disclosure]

### [Technical Problem]

An object of the present invention is to provide a deoxycholic acid-peptide conjugate having improved solubility in water and improved adipogenesis inhibitory activity and lipolysis activity compared to a single substance.

Another object of the present invention is to provide a composition for inhibiting adipogenesis or promoting lipolysis.

Yet another object of the present invention is to provide a method for inhibiting adipogenesis or promoting lipolysis.

Yet another object of the present invention is to provide a deoxycholic acid-peptide conjugate used for inhibiting adipogenesis or promoting lipolysis.

Yet another object of the present invention is to provide a composition for preventing, treating or ameliorating obesity or obesity-related diseases.

Yet another object of the present invention is to provide a method for preventing or treating obesity or obesity-related diseases.

Yet another object of the present invention is to provide a deoxycholic acid-peptide conjugate used for preventing or treating obesity or obesity-related diseases.

### [Technical Solution]

An aspect of the present invention provides a deoxycholic acid-peptide conjugate in which a peptide consisting of an amino acid sequence of SEQ ID NO: 1 and deoxycholic acid are conjugated.

Another aspect of the present invention provides a composition for inhibiting adipogenesis or promoting lipolysis comprising a deoxycholic acid-peptide conjugate as an active ingredient.

Yet another aspect of the present invention provides a method for inhibiting adipogenesis or promoting lipolysis comprising administering the deoxycholic acid-peptide conjugate of claim 1 to a subject.

Yet another aspect of the present invention provides a deoxycholic acid-peptide conjugate used for inhibiting adipogenesis or promoting lipolysis.

Yet another aspect of the present invention provides a composition for preventing, treating or ameliorating obesity or obesity-related diseases comprising a deoxycholic acid-peptide conjugate as an active ingredient.

Yet another aspect of the present invention provides a method for preventing or treating obesity or obesity-related diseases comprising administering the deoxycholic acid-peptide conjugate of claim 1 to a subject.

Yet another object of the present invention is to provide a deoxycholic acid-peptide conjugate used for preventing or treating obesity or obesity-related diseases.

### [Advantageous Effects]

According to the present invention, the deoxycholic acid-peptide conjugate has an effect of inhibiting the lipid production and accumulation in adipocytes and decomposing fat and thus can be used for preventing, ameliorating or treating obesity.

However, the effects of the present invention are not limited to the above-mentioned effects, and other effects not mentioned will be clearly understood by those skilled in the art from the following description.

### [Description of Drawings]

FIG. 1 illustrates NMR analysis data for a conjugate of a peptide of SEQ ID NO: 1 and deoxycholic acid of the present invention.
FIG. 2 is a photograph showing results of confirming solubility in water of deoxycholic acid and a deoxycholic acid-peptide conjugate.
FIG. 3A is a diagram illustrating a result of confirming an effect of inhibiting differentiation of adipocytes and intracellular lipid accumulation by the deoxycholic acid-peptide conjugate through intracellular fat staining.
FIG. 3B is a diagram illustrating a result of confirming an increased fat accumulation inhibitory effect of the deoxycholic acid-peptide conjugate through intracellular fat staining.
FIG. 4A is a diagram illustrating a result of confirming an effect of promoting intracellular lipolysis by the deoxycholic acid-peptide conjugate through a release amount of glycerol released in adipocytes.
FIG. 4B is a diagram illustrating a result of confirming an effect of promoting intracellular lipolysis by the deoxycholic acid-peptide conjugate through a decreased size of adipocytes in adipose tissue.
FIG. 4C is a diagram illustrating a result of confirming an increased lipolysis effect of the deoxycholic acid-peptide conjugate through a release amount of glycerol released in adipocytes.
FIG. 5 is a diagram illustrating a result of confirming that the expression of adiponectin and leptin genes related to intracellular fat metabolism has been increased by the deoxycholic acid-peptide conjugate.
FIG. 6A is a diagram illustrating a result of confirming that the expression of lipolysis-related cell signaling pathway genes AMPKa1, HSL, and PGCLa has been increased by the deoxycholic acid-peptide conjugate in adipocytes.
FIG. 6B is a diagram illustrating a result of confirming that the expression of lipolysis-related cell signaling pathway genes HSL and PLIN has been increased by the deoxycholic acid-peptide conjugate in adipose tissue.
FIG. 7 is a diagram illustrating a result of confirming that the expression of adipogenesis-related cell signaling pathway genes ACCα, FAS and PPARγ has been decreased by the deoxycholic acid-peptide conjugate in adipose tissue.
FIG. 8 is a diagram illustrating a result of confirming that the expression of thermogenic genes UCP1, PRDM and Cidea of brown adipocytes has been increased in adipose tissue.
FIG. 9 is a diagram illustrating a result of confirming that the expression of an IL-15 gene related to the inhibition of lipid accumulation has been increased in muscle cells.

### [Best Mode]

Hereinafter, the present invention will be described in detail.

One aspect of the present invention provides a peptide comprising an amino acid sequence of SEQ ID NO: 1 and an active substance-peptide conjugate in which an active substance is conjugated to the peptide.

The synthesis of the peptide of the present invention may be performed using, for example, devices or genetic engineering techniques. In the case of synthesis using the devices, desired peptides may be synthesized using an Fmoc solid-phase method in an automatic peptide synthesizer.

In a specific exemplary embodiment, the peptide including the amino acid sequence of SEQ ID NO: 1 of the present invention was synthesized and identified using the Fmoc solid-phase method, and was selected by verifying the efficacy of the identified peptide.

In a specific exemplary embodiment, in order to verify the efficacy of the identified peptide, a peptide having anti-obesity activity was selected through validation of i) an adipogenesis inhibitory effect and ii) a lipolysis effect.

Furthermore, an active substance-peptide conjugate of the present invention may be prepared by conjugating the peptide selected through the process with the active substance. In a specific exemplary embodiment, the active substance was selected by verifying the efficacy of the active substance-peptide conjugates prepared by being chemically conjugated with the peptide.

In the present specification, the term `peptide' refers to a linear molecule consisting of amino acid residues, and specifically, the peptide of the present invention may include the amino acid sequence represented by SEQ ID NO: 1.

The `peptide' may be variants or fragments of amino acids having different sequences by deletion, insertion, substitution or a combination thereof of amino acid residues within a range that does not affect a function. Amino acid exchange that does not entirely alter the activity of the peptide is known in the art. In some cases, the amino acid exchange may be modified by phosphorylation, sulfation, acrylation, glycosylation, methylation, farnesylation, and the like. Accordingly, the present invention includes peptides having an amino acid sequence substantially identical to the peptide having the amino acid sequence of SEQ ID NO: 1, and variants or active fragments thereof. The substantially identical proteins refer to amino acid sequences having at least 75%, preferably at least 80%, for example, at least 85%, at least 90%, at least 95%, at least 98%, or at least 99% of sequence homology with the amino acid sequence of SEQ ID NO: 1, but are not limited thereto, and amino acid sequences having 75% or more homology and the same activity are included in the scope of the present invention. In addition, the peptide of the present invention may further include an amino acid sequence prepared for a specific purpose of increasing a targeting sequence, a tag, labeled residues, half-life or peptide stability.

In addition, in order to obtain better chemical stability, enhanced pharmacological properties (half-life, absorbency, titer, efficacy, etc.), modified specificity (e.g., a wide biological activity spectrum), and reduced antigenicity, a protective group may bind to an N- or C-terminus of the peptide. For example, the protective group may be an acetyl group, a fluorenylmethoxy carbonyl group, a formyl group, a palmitoyl group, a myristyl group, a stearyl group, or polyethylene glycol (PEG), but may include any ingredient that may enhance the modification of the peptide, particularly the stability of the peptide, without limitation. The 'stability' is used to the meaning including not only in-vivo stability that protects the peptide of the present invention from the attack of protein cleavage enzymes in vivo, but also storage stability (e.g., room-temperature storage stability).

In the present specification, the `active substance' may be bile acid, specifically, may be preferably used with bile acid selected from the group consisting of cholic acid, 5β-cholanic acid, chenodeoxycholic acid, glycocholic acid, taurocholic acid, deoxycholic acid, lithocholic acid, 7-oxo-lithocholic acid, etc., and more specifically, the active substance may be deoxycholic acid.

The 'deoxycholic acid' of the present invention is a compound having an IUPAC name of 3,12-dihydroxycholan-24-oic acid and a molecular weight of 392.58. In general, the deoxycholic acid is a type of bile acid that decomposes fats and destroys adipocytes, and is used in injections for ameliorating fat under the chin. A structure of the deoxycholic acid is shown in Chemical Formula 1 below.

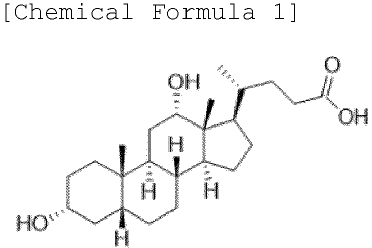

In the active substance-peptide conjugate of the present invention, when the active substance is deoxycholic acid, the peptide conjugated with the deoxycholic acid is referred to as a 'deoxycholic acid-peptide conjugate (DA-peptide conjugate)'.

Specifically, the present invention provides a deoxycholic acid-peptide conjugate in which deoxycholic acid is conjugated with a peptide consisting of an amino acid sequence of SEQ ID NO: 1.

The deoxycholic acid may be conjugated with an N-terminus, C-terminus or side chain of the peptide. Specifically, a carboxyl group of the deoxycholic acid may be peptide-bound with the N-terminus of the peptide.

An embodiment of the deoxycholic acid-peptide conjugate may be represented by Chemical Formula 2 below.

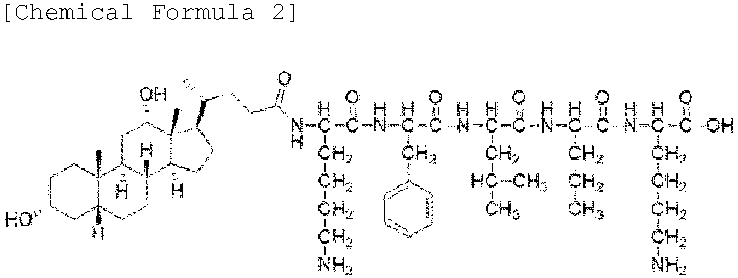

In a specific exemplary embodiment of the present invention, the deoxycholic acid-peptide conjugate is administered into adipocytes and adipose tissue to inhibit a signaling pathway of adipogenesis and inhibit fat production and accumulation, thereby preventing, ameliorating, or treating obesity.

In addition, the deoxycholic acid-peptide conjugate of the present invention may activate a signaling pathway of lipolysis to promote the lipolysis in adipocytes and adipose tissue and reduce the sizes of the adipocytes, thereby preventing, ameliorating, or treating obesity.

In addition, the deoxycholic acid-peptide conjugate of the present invention has high solubility in water, and has a fat accumulation inhibitory effect and a lipolysis effect significantly improved as compared with a deoxycholic acid or peptide single substance to reduce the used amount of deoxycholic acid, thereby preventing side effects caused by the use of a high dose of deoxycholic acid.

Therefore, the deoxycholic acid-peptide conjugate having the activity may be used as an active ingredient of the composition for preventing, treating, or ameliorating obesity or obesity-related diseases.

Therefore, another aspect of the present invention provides a composition for preventing, treating, or ameliorating obesity or obesity-related diseases including a deoxycholic acid-peptide conjugate in which deoxycholic acid is conjugated with a peptide including an amino acid sequence of SEQ ID NO: 1.

Yet another aspect of the present invention provides a composition for inhibiting adipogenesis or promoting lipolysis including a deoxycholic acid-peptide conjugate in which deoxycholic acid is conjugated with a peptide including an amino acid sequence of SEQ ID NO: 1.

In addition, the present invention provides a method for inhibiting adipogenesis or promoting lipolysis including administering, to a subject, a deoxycholic acid-peptide conjugate in which deoxycholic acid is conjugated with a peptide including an amino acid sequence of SEQ ID NO: 1.

In addition, the present invention provides a deoxycholic acid-peptide conjugate in which deoxycholic acid is conjugated with a peptide including an amino acid sequence of SEQ ID NO: 1, used for inhibiting adipogenesis or promoting lipolysis.

Yet another aspect of the present invention provides a pharmaceutical composition for preventing or treating obesity or obesity-related diseases including a deoxycholic acid-peptide conjugate in which deoxycholic acid is conjugated with a peptide including an amino acid sequence of SEQ ID NO: 1.

In addition, the present invention provides a method for preventing or treating obesity or obesity-related diseases including administering, to a subject, a deoxycholic acid-peptide conjugate in which deoxycholic acid is conjugated with a peptide including an amino acid sequence of SEQ ID NO: 1.

In addition, the present invention provides a deoxycholic acid-peptide conjugate in which deoxycholic acid is conjugated with a peptide including an amino acid sequence of SEQ ID NO: 1, used for preventing or treating obesity or obesity-related diseases.

In the present specification, the `obesity' means a condition or disease in which body fat is excessively accumulated in the body due to energy imbalance. The deoxycholic acid-peptide conjugate of the present invention not only significantly increases the expression of a fat oxidation promoting gene and a protein encoded by the gene, and a lipolysis promoting gene and a protein encoded by the gene, but also decreases the expression of a gene involved in the accumulation of adipocytes to control the imbalance or deviate from the condition having excess body fat, thereby preventing or treating the obesity.

In the present specification, the `prevention or treatment of obesity' may mean reducing fat deposition, and the 'fat deposition' may mean a condition selected from the group consisting of, for example, obesity, fat redistribution syndrome, lower eyelid fat hernia, lipoma, Derkham's disease, lipodystrophy, visceral adiposity, breast enlargement, hyperobesity, diffused body fat around the flanks and arms, and fat deposition associated with cellulite.

In addition, in the present specification, the `obesity-related disease' is a disease that may be caused by a condition having excessive body fat due to energy imbalance, and symptoms caused from the obesity-related diseases may be prevented, alleviated or ameliorated through the prevention or treatment of obesity. Specifically, the obesity-related diseases may include hypertension, diabetes, increased plasma insulin concentration, insulin resistance, hyperlipidemia, metabolic syndromes, insulin resistant syndromes, obesity-related gastroesophageal reflux, arteriosclerosis, hypercholesterolemia, hyperuricemia, lower back pain, cardiac hypertrophy and left ventricular hypertrophy, lipodystrophy, non-alcoholic steatohepatitis, cardiovascular disease, polycystic ovarian syndrome, etc.

The composition for preventing or treating obesity or obesity-related diseases of the present invention may be formulated and used in the form of oral formulations such as powders, granules, tablets, capsules, suspensions, emulsions, syrups, aerosols, external preparations, suppositories, and sterile injections according to conventional methods, and may include suitable carriers, excipients or diluents commonly used in the preparation of the pharmaceutical composition for formulation.

The pharmaceutically acceptable carrier may include lactose, dextrose, sucrose, sorbitol, mannitol, starch, acacia rubber, calcium phosphate, alginate, gelatin, calcium silicate, microcrystalline cellulose, polyvinylpyrrolidone, cellulose, water, syrup, methylcellulose, methylhydroxybenzoate, propylhydroxybenzoate, talc, magnesium stearate, and mineral oil, which are generally used in preparation.

The pharmaceutical composition may further include a lubricant, a wetting agent, a sweetening agent, a flavoring agent, an emulsifying agent, a suspending agent, a preservative, and the like, in addition to the ingredients.

The pharmaceutical composition may be administered orally or parenterally (e.g., applied intramuscularly, intravenously, intraperitoneally, subcutaneously, intradermally, or topically) according to a desired method, and a dose thereof varies depending on the condition and weight of a patient, a degree of disease, a drug form, and route and time of administration, but may be appropriately selected by those skilled in the art.

When the pharmaceutical composition of the present invention is used as an injectable formulation, the pharmaceutical composition may be used to remove localized fat deposition.

The localized fat may be localized in a target area selected from the group consisting of the abdomen, under the eyes, under the chin, under the arms, buttocks, thighs, calves, back, thighs, and bra line.

The pharmaceutical composition of the present invention is administered in a pharmaceutically effective dose. In the present invention, the `pharmaceutically effective dose' refers to a sufficient amount to treat the diseases at a reasonable benefit/risk ratio applicable to medical treatment, and an effective dose level may be determined according to factors including the type and severity of disease of a patient, activity of a drug, sensitivity to a drug, a time of administration, a route of administration, an emission rate, duration of treatment, and simultaneously used drugs, and other factors well-known in the medical field. The pharmaceutical composition may be administered as an individual therapeutic agent or in combination with other therapeutic agents, and may be administered simultaneously, separately, or sequentially with conventional therapeutic agents, and may be administered singly or multiply. It is important to administer an amount capable of obtaining a maximum effect with a minimal amount without side effects by considering all the elements, which may be easily determined by those skilled in the art.

The effective dose of the pharmaceutical composition may vary depending on the age, sex, condition, and body weight of a patient, absorbance of an active ingredient in vivo, an inactivation rate, an excretion rate, a disease type, and drugs to be used in combination, and may be increased or decreased according to a route of administration, the severity of obesity, sex, body weight, age, and the like. For example, the pharmaceutical composition may be administered at about 0.0001 µg to 500 mg, preferably 0.01 µg to 100 mg per 1 kg of patient's body weight per day.

In a specific exemplary embodiment, when the deoxycholic acid-peptide conjugate of the present invention is administered to adipocytes or adipose tissue, as compared to a case of administering the deoxycholic acid or peptide alone, it was confirmed that the fat accumulation inhibitory effect and the lipolysis effect were significantly increased. Therefore, when the deoxycholic acid-peptide conjugate is used, the usage amount may be reduced through a synergistic effect, thereby securing safety by using a minimum amount of deoxycholic acid and minimizing side effects caused by deoxycholic acid. The 'subject' may be a subject in need of administration of the deoxycholic acid-peptide conjugate of the present invention. The subject in need of the administration may include a subject who has been diagnosed with obesity or obesity-related diseases, a subject who has developed obesity or obesity-related symptoms, and a subject who wants to be administered for preventing the development of the disease or symptoms or ameliorating the health.

The 'administration' means providing a predetermined substance to a patient by any suitable method, and the administration route of the deoxycholic acid-peptide conjugate of the present invention may be administered orally or parenterally through all general routes as long as the deoxycholic acid-peptide conjugate may reach a target tissue. In addition, the deoxycholic acid-peptide conjugate may be administered by any device capable of moving the active substance to a target cell.

In yet another aspect, the present invention provides a quasi-drug composition for preventing or ameliorating obesity including a deoxycholic acid-peptide conjugate in which deoxycholic acid is conjugated with a peptide including an amino acid sequence of SEQ ID NO: 1.

Detailed contents of the peptide, the deoxycholic acid and the obesity are as described above.

When the composition of the present invention is used as the quasi-drug composition, the deoxycholic acid-peptide conjugate may be added as it is or used together with other quasi-drug ingredients, and may be appropriately used according to a conventional method. The mixed amount of the active ingredients may be suitably determined according to a purpose of use (prevention, health, or therapeutic treatment). According to one exemplary embodiment of the present invention, the quasi-drug composition of the present invention may be a disinfectant cleaner, a toothpaste, a shower foam, Garglin, wet tissue, detergent soap, a hand wash, a humidifier filler, a mask, an ointment, or a filter filler.

In yet another aspect, the present invention provides a cosmetic composition for preventing or ameliorating obesity including a deoxycholic acid-peptide conjugate in which deoxycholic acid is conjugated with a peptide including an amino acid sequence of SEQ ID NO: 1.

Detailed contents of the peptide, the deoxycholic acid and the obesity are as described above.

The cosmetic composition of the present invention may be prepared in any formulation commonly prepared in the art, and may be formulated by, for example, a solution, a suspension, an emulsion, a paste, a gel, a cream, a lotion, a powder, a soap, a surfactant-containing cleansing, oil, a powder foundation, an emulsion foundation, a wax foundation, a spray, and the like, but is not limited thereto.

The cosmetic composition may be prepared in various forms, such as softening lotion, nutrient lotion, nutrient cream, massage cream, essence, eye cream, cleansing cream, cleansing foam, cleansing water, pack, spray, powder, hair tonic, hair cream, hair lotion, hair shampoo, and hair rinse, hair conditioners, hair sprays, hair aerosols, pomades, solutions such as gels, sol gels, emulsions, oils, waxes, aerosols, etc., but is not limited thereto.

The cosmetic composition of the present invention may include other additives such as excipients and carriers in addition to a pentapeptide consisting of an amino acid sequence of KFLIK, and can be applied and mixed with common ingredients mixed in general skin cosmetics as needed.

When the form of the formulation of the cosmetic composition is the paste, cream, or gel, as the carrier ingredient, animal oils, vegetable oils, wax, paraffin, starch, tragacanth, cellulose derivatives, polyethylene glycol, silicone, bentonite, silica, talc, zinc oxide or the like may be used.

When the form of the formulation of the cosmetic composition is the powder or spray, as the carrier ingredient, lactose, talc, silica, aluminum hydroxide, calcium silicate or polyamide powder may be used, and particularly, in the case of the spray, additionally, a propellant such as chlorofluorohydrocarbon, propane/butane or dimethyl ether may be included, but is not limited thereto.

When the form of the formulation of the cosmetic composition is the solution or emulsion, as the carrier ingredient, a solvent, a solubilizing agent or an emulsifying agent may be used. For example, the carrier ingredient may be used with water, ethanol, isopropanol, ethyl carbonate, ethyl acetate, benzyl alcohol, benzyl benzoate, propylene glycol, 1,3-butyl glycol oil, glycerol aliphatic ester, polyethylene glycol or fatty acid ester of sorbitan.

When the form of the formulation of the cosmetic composition is the suspension, as the carrier ingredient, a liquid diluent such as water, ethanol or propylene glycol, a suspension such as ethoxylated isostearyl alcohol, polyoxyethylene sorbitol ester and polyoxyethylene sorbitan ester, microcrystalline cellulose, aluminum metahydroxide, bentonite, agar, tragacanth, or the like may be used.

When the formulation of the cosmetic composition is the surfactant-containing cleansing, as the carrier ingredient, aliphatic alcohol sulfate, aliphatic alcohol ether sulfate, sulfosuccinic acid monoester, isethionate, imidazolinium derivatives, methyltaurate, sarcosinate, fatty acid amide ether sulfate, alkylamido betaine, aliphatic alcohol, fatty acid glyceride, fatty acid diethanolamide, vegetable oil, lanolin derivatives, ethoxylated glycerol fatty acid ester, or the like may be used.

When the formulation of the cosmetic composition is the hair shampoo, base ingredients for forming the shampoo, such as a thickener, a surfactant, a viscosity modifier, a moisturizer, a pH modifier, a preservative, and essential oil, may be mixed with a deoxycholic acid-peptide conjugate of the present invention. CDE may be used as the thickener, LES as an anionic surfactant and coco betaine as an amphoteric surfactant may be used as the surfactant, poly quarter may be used as the viscosity modifier, glycerin may be used as the moisturizer, and citric acid and sodium hydroxide may be used as the pH modifier. Grapefruit extract and the like may be used as the preservative, and in addition, essential oils such as cedarwood, peppermint, and rosemary, silk amino acids, pentaol, or vitamin E may be added.

The ingredients included in the cosmetic composition may further include ingredients commonly used in cosmetic compositions, for example, conventional adjuvants such as antioxidants, stabilizers, solubilizers, vitamins, pigments and spices in addition to the deoxycholic acid-peptide conjugate of the present invention as the active ingredient and the carrier ingredient.

As described above, the deoxycholic acid-peptide conjugate of the present invention has effects of inhibiting difference into adipocytes from preadipocytes, inhibiting lipid accumulation in the adipocytes, and promoting lipolysis and thus can be used as a raw material of the pharmaceutical or cosmetic composition for the purpose of preventing, ameliorating or treating obesity or obesity-related diseases.

Hereinafter, the present invention will be described in detail by Examples and Experimental Examples.

However, the following Examples and Experimental Examples are just illustrative of the present invention, and the contents of the present invention are not limited to the following Examples and Experimental Examples.

### [Example 1]

### Synthesis of peptide including amino acid sequence of SEQ ID NO: 1

A peptide of the present invention was synthesized by a known method, an amino acid sequence of a novel peptide used in the present invention was `Lys Phe Leu Ile Lys (SEQ ID NO: 1)', and a molecular weight of the peptide including the amino acid sequence of SEQ ID NO: 1 was measured with a molecular weight meter, and as a result, it was confirmed that the molecular weight corresponded to 647.4 Da.

### [Example 2]

### Preparation of deoxycholic acid-peptide conjugate

A peptide (500 mmol) of SEQ ID NO: 1 and 1,000 mL of dimethylformamide (DMF) were added into a peptide reactor, 393 g of deoxycholic acid (1,000 mmol, 2 equiv.), 135 g of 1-HOBt (1000 mmol, 2 equiv.), and 379 g of HBTU (1000 mmol, 2 equiv.) were added with 500 ml of DMF and reacted at room temperature for 30 minutes. Thereafter, 261 mL (194 g, 1500 mmol, 3 equiv.) of diisopropylethylamine (DIPEA) was further added and reacted at room temperature for 1 hour. After the reaction was completed, a deoxycholic acid-peptide resin was filtered and washed, and then dried in a vacuum oven. Thereafter, the deoxycholic acid-peptide resin was added with 20 L of diethyl ether, crystallized, filtered and separated, and then purified to prepare a deoxycholic acid-peptide conjugate.

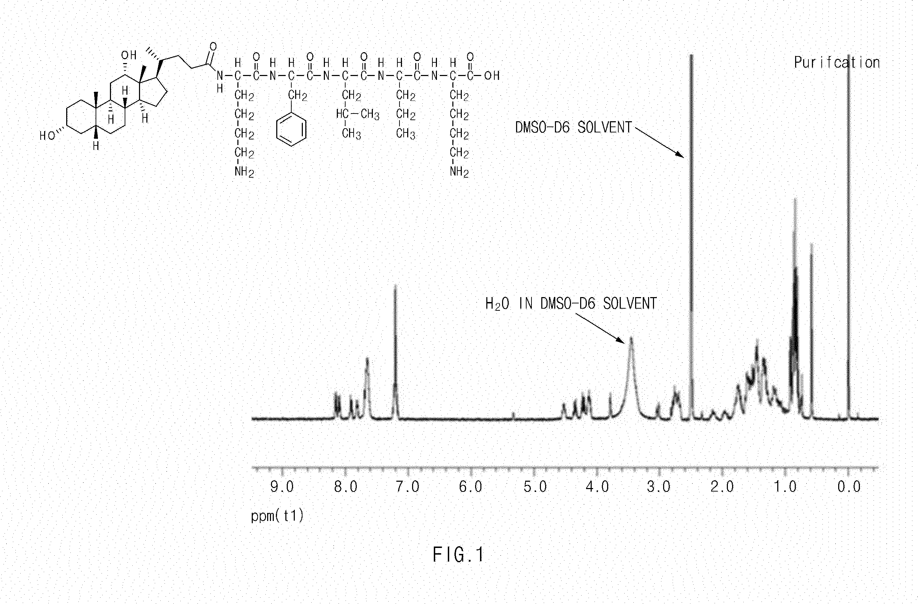

As a result of measuring the molecular weight of the deoxycholic acid-peptide conjugate using the molecular weight meter, it was confirmed that the molecular weight corresponded to 1022.4 Da. In addition, a result of analyzing the prepared deoxycholic acid-peptide conjugate by ¹H-NMR was illustrated in FIG. 1. In the ¹H-NMR analysis spectrum, a peak of the peptide was shown in addition to a peak of deoxycholic acid, and as a result, it was clearly confirmed that the deoxycholic acid-peptide conjugate was synthesized.

### [Preparation Example 1]

### Preparation of preadipocytes and induction of differentiation into adipocytes

3T3-L1 cells as preadipocytes were purchased from the American Type Culture Collection (ATCC) and used in an experiment. 3T3-L1 was a cell line widely used to study the metabolic process of adipocytes, and as the differentiation of the cells was activated, fat accumulation in adipocytes was activated to induce obesity. Accordingly, when the cells were treated with a substance considered to have an anti-obesity effect, it could be seen that the substance had a great anti-obesity effect as the differentiation of the cells was smaller.

The preadipocytes 3T3-L1 were cultured in a DMEM containing 2% fetal bovine serum (FBS) under conditions of 37°C and 5% CO₂. The preadipocytes 3T3-L1 were dispensed in a 12-well plate at a density of 1 × 10⁵ cells/well, and then further maintained for 2 days at a 100% confluency point. The preadipocytes were cultured for 2 days in a DMEM medium (hereinafter referred to as a 'differentiation medium') containing a differentiation ingredient (including 10 µg/ml insulin, 0.1 µM dexamethasone and 0.5 mM IBMX) and 10% FBS to induce the differentiation into adipocytes. After 48 hours of culture, the preadipocytes were cultured for 2 days in 10% FBS DMEM (hereinafter referred to as a `culture medium') containing 1 µg/ml insulin. Thereafter, the culture medium was replaced with a 10% FBS DMEM culture medium every 2 days. During the induction of adipocytes differentiation, depending on a purpose of the experiment, a deoxycholic acid-peptide conjugate was treated or not in each culture medium at a concentration of 1 µM or 10 µM, and the differentiation degree of adipocytes was observed on day 7, when the differentiation was completed.

### [Preparation Example 2]

### Collection and culture of adipose tissue

Adipose tissue was collected from the abdomen of 9-week-old BL/6 mice, placed in a 100 mm × 15 mm culture dish, and then cultured in a culture medium overnight. The adipose tissue was divided to have the same weight, transferred to a 24-well plate, and then used in the following Experimental Examples.

### [Experimental Example 1]

### Confirmation of solubility of deoxycholic acid-peptide conjugate

Solubilities of the deoxycholic acid-peptide conjugate prepared in Example 2 and deoxycholic acid were visually observed and compared.

Specifically, 90 µL of distilled water was added to an E. tube containing 1 mg each of the deoxycholic acid-peptide conjugate and the deoxycholic acid, and dissolution was confirmed.

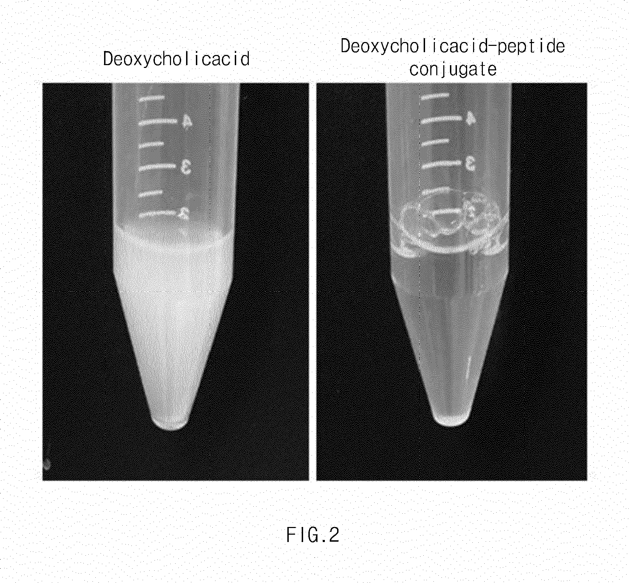

As a result, as illustrated in FIG. 2, in contrast to the fact that deoxycholic acid was hardly dissolved in water, it was confirmed that the deoxycholic acid-peptide conjugate of the present invention were completely dissolved in water.

Through the results, it can be seen that in the deoxycholic acid-peptide conjugate of the present invention, the peptide was conjugated with the deoxycholic acid, so that the water solubility was improved compared to a case where the deoxycholic acid was present alone.

### [Experimental Example 2]

### Fat accumulation inhibitory effect of deoxycholic acid-peptide conjugate

### 2-1. Oil red O staining and analysis

Oil red O staining was performed to confirm whether the deoxycholic acid-peptide conjugate of the present invention had an effect of inhibiting the fat accumulation in adipocytes.

Specifically, the degree of differentiation of adipocytes induced in Preparation Example 1 was first confirmed under a microscope through oil red O staining, and for the staining degree of adipocytes, the degree of fat accumulated in the cells was calculated by measuring absorbance with a spectrophotometer at a wavelength of 510 nm. A group treated with a β3 adrenergic receptor aonist CL316243 or TNF-α was used as a positive control group.

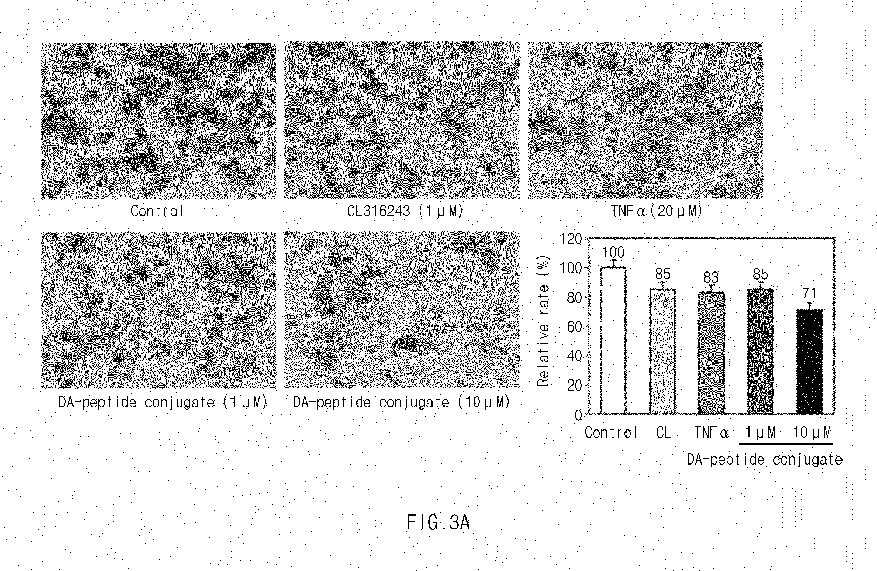

As a result, as illustrated in FIG. 3A, when 1 µM of the deoxycholic acid-peptide conjugate was treated, the fat staining in adipocytes was reduced at the same level as the positive control group.

As a result, it can be seen that the deoxycholic acid-peptide conjugate may inhibit differentiation into adipocytes and adipogenesis to inhibit lipid accumulation in adipocytes.

### 2-2. Confirmation of synergistic fat accumulation inhibitory effect by deoxycholic acid-peptide

In order to compare the fat accumulation inhibitory effects in the case of treating the deoxycholic acid-peptide conjugate and the case of treating the deoxycholic acid or the peptide of SEQ ID NO: 1 alone, the degree of differentiation of adipocytes and the degree of lipid accumulation were measured in the same manner as in Experimental Example 1-1. A group treated with TNF-α was used as a positive control group.

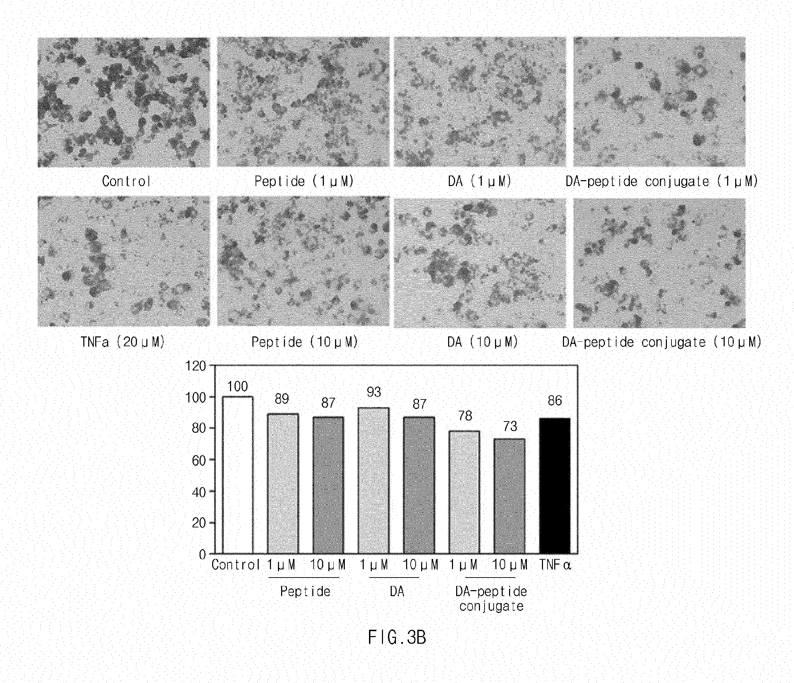

As a result, as illustrated in FIG. 3B, when the peptide of SEQ ID NO: 1 and the deoxycholic acid were treated at 1 µM each, the lipid accumulation amounts were 89% and 93%, respectively, but when the deoxycholic acid-peptide conjugate was treated at the same concentration, it was confirmed that the lipid accumulation amount was 78%.

As a result, it can be seen that in the case of treating the same concentration of deoxycholic acid-peptide conjugate as compared with the case of treating the peptide of SEQ ID NO: 1 or deoxycholic acid alone, the degrees of differentiation into adipocytes and inhibition of fat accumulation are greatly increased. In addition, it can be seen that even when a small amount of deoxycholic acid-peptide conjugate is used, an effect equal to or higher than that of a single compound can be exhibited.

### [Experimental Example 3]

### Lipolysis effect of deoxycholic acid-peptide conjugate

### 3-1. Measurement of amount of glycerol as lipolysis product in adipose tissue

Neutral fat stored in adipose tissue was decomposed into fatty acids and glycerol for energy production or cell signaling. Therefore, the lipolysis effect of neutral fat accumulated in the adipose tissue was confirmed by measuring the release amount of free glycerol.

Specifically, in female mice fed with a high-fat diet (Rodent Diet with 60% Kcal Fat, Research Diets, USA), a fat pad, which was white adipose tissue, was isolated, and each 400 mg of adipose tissue per well was placed in a 12-well plate, and then cultured for 48 hours in a culture medium (DMEM media serum free) to be stabilized. During the culture, the peptide conjugate was treated at concentrations of 1 µM, 10 µM, and 100 µM, and as a positive control group, 20 nM of TNFα was treated and cultured for 48 hours to obtain a supernatant, and the amount of glycerol was measured using a glycerol colorimetric assay kit (Cayman chemical, USA).

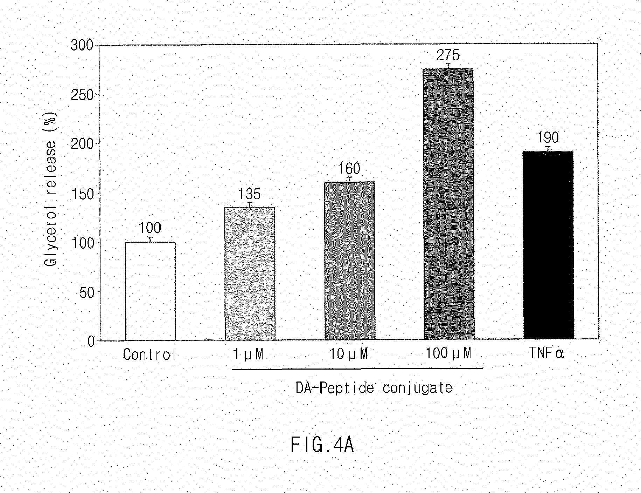

As a result, as illustrated in FIG. 4A, it was confirmed that when the deoxycholic acid-peptide conjugate was treated compared to the control group, the amount of glycerol released while the fat was decomposed was increased in a concentration-dependent manner of the deoxycholic acid-peptide conjugate.

As a result, it can be seen that the deoxycholic acid-peptide conjugate of the present invention has the activity of decomposing neutral fat accumulated in adipose tissue.

### 3-2. Confirmation of reduction in size of adipocytes in adipose tissue

In order to confirm the lipolysis effect in adipose tissue by the deoxycholic acid-peptide conjugate, H&E staining was performed on the adipose tissue obtained in Preparation Example 2 to confirm the sizes of adipocytes.

Specifically, the adipose tissue was cut, attached to a paraffin slide, and dried at 50°C. The slide attached with the dried adipose tissue was placed in Xylene 1, 2, and 3 for 5 minutes each, and the paraffin was removed. Thereafter, the slide was put into 100% ethanol for 2 minutes, and then sequentially put into 90%, 80%, and 70% ethanol to be re-hydrated. The re-hydrated slide was stained in a hematoxylin solution, washed, and then stained with Eosin Y. Thereafter, the slide was sequentially put into 90%, 80%, and 70% ethanol, and then put into Xylene 3, 2, and 1, and then mounted with a xylene-based mounting medium to prevent air bubbles from entering the tissue, and then dried. Then, the dried slide was observed under an optical microscope.

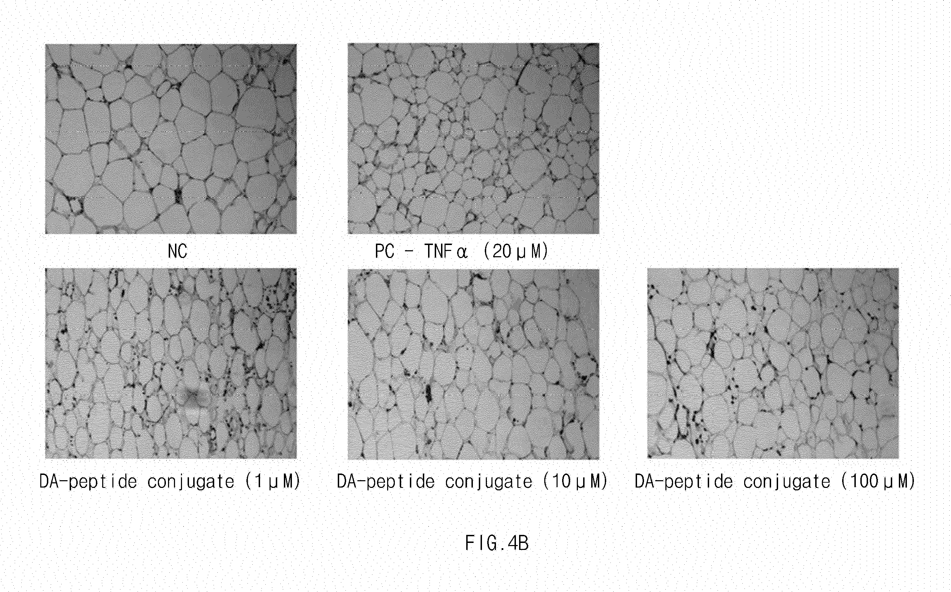

As a result, as illustrated in FIG. 4B, it was confirmed that when the adipose tissue was treated with the deoxycholic acid-peptide conjugate, the sizes of adipocytes were decreased compared to the control group.

As a result, it can be seen that the fat that had been accumulated in the cells was decomposed by the deoxycholic acid-peptide conjugate of the present invention.

### 3-3. Confirmation of synergistic lipolysis effect of deoxycholic acid-peptide conjugate

In order to compare the lipolysis effects in the case of treating the deoxycholic acid-peptide conjugate and the case of treating the deoxycholic acid or the peptide of SEQ ID NO: 1 alone, the amount of glycerol released from the adipose tissue was measured in the same manner as in Experimental Example 3-1. A group treated with TNF-α was used as a positive control group.

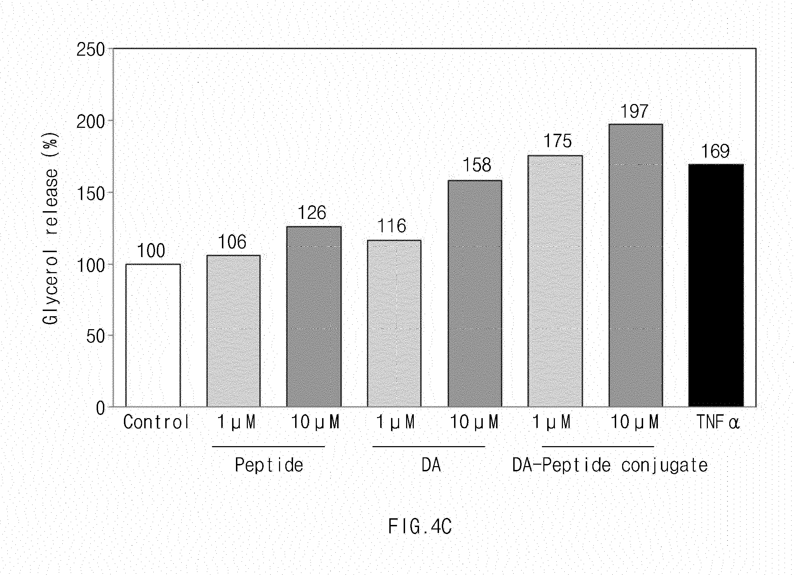

As a result, as illustrated in FIG. 4C, it was confirmed that when the peptide of SEQ ID NO: 1 and the deoxycholic acid were treated at 1 µM each, the glycerol release amounts were 106% and 116%, respectively, but when the deoxycholic acid-peptide conjugate was treated at the same concentration, the glycerol release amount was 175%.

As a result, it can be seen that in the case of treating the same concentration of deoxycholic acid-peptide conjugate as compared with the case of treating the peptide of SEQ ID NO: 1 or deoxycholic acid alone, the glycerol release amount according to lipolysis is greatly increased. In addition, it can be seen that even when a small amount of deoxycholic acid-peptide conjugate is used, an effect equal to or higher than that of a single compound can be exhibited.

### [Experimental Example 4]

### Confirmation of expression changes of genes related to adipogenesis and lipolysis

### 4-1. RT-PCT analysis

The adipocytes obtained in Preparation Example 1 or the adipose tissue obtained in Preparation Example 2 were treated with the deoxycholic acid-peptide conjugate of the present invention, cultured, and then recovered, and RNA was extracted with Trizol (Thermo Fisher Scientific, USA). From the extracted RNA, complementary DNA to RNA was obtained using TOPScript^{™} RT DryMIX (enzynomics, Korea) and then polymerase chain reaction (PCR) was performed using TOPsimple ^{™} DryMIX-nTaq (enzynomics, Korea). Thereafter, PCR products were loaded on a 1.5% agarose gel to compare the mRNA expression levels of the growth factors. A GAPDH gene was used as an internal control group, and primers used in the experiment were shown in Table 1.

**[Table 1]**

| Gene | Primer | Sequence (5' → 3') | SEQ ID NO: |
|---|---|---|---|
| Leptin | F | GGATCAGGTTTTGTGGTGCT | 2 |
| | R | TTGTGGCCCATAAAGTCCTC | 3 |
| Adiponectin | F | TCCTGCTTTGGTCCCTCCAC | 4 |
| | R | TCTCCAGCCCCACACTGAAC | 5 |
| AMPKα | F | TCACCGGACATAAAGTGGCT | 6 |
| | R | TGATGATGTGAGGGTGCCTG | 7 |
| HSL | F | GGACACACACACACCTG | 8 |
| | R | CCCTTTCGCAGCAACTTTAG | 9 |
| PDE3b | F | TGATTGACCCAAGCCTTCCC | 10 |
| | R | TCTTACCACTGCTGCGATCC | 11 |
| PGCLa | F | ATGTGCAGCCAAGACTCTGTA | 12 |
| | R | CGCTACACCACTTCAATCCAC | 13 |
| PLIN | F | AAGGATCCTGCACCTCACAC | 14 |
| | R | CCTCTGCTGAAGGGTTATCG | 15 |
| ACCα | F | ACCTTACTGCCATCCCATGTGCTA | 16 |
| | R | GTGCCTGATGATCGCACGAACAAA | 17 |
| FAS | F | TGCTGGCACTACAGAATGC | 18 |
| | R | AACAGCCTCAGAGCGACAAT | 19 |
| PPARγ | F | ATGGGTGAAACTCTGGGAGA | 20 |
| | R | GAGCTGATTCCGAAGTTGGT | 21 |
| GAPDH | F | GTGATGGCATGGACTGTGGT | 22 |
| | R | GGAGCCAAAAGGGTCATCAT | 23 |

### 4-2. Confirmation of increased expression of genes related to fat metabolism using adipocytes

In order to confirm whether the deoxycholic acid-peptide conjugate promoted fat metabolism, mRNA expression levels of adiponectin and leptin genes related to fat metabolism were confirmed.

Specifically, the adipocytes obtained in Preparation Example 1 were treated with 1 µM and 10 µM of the deoxycholic acid-peptide conjugate of the present invention, and cultured for 1 hour, 3 hours, and 6 hours. After the cells were recovered and the polymerase chain reaction was performed by the method described in Experimental Example 4-1, the expression products were loaded on an agarose gel to confirm the mRNA expression levels of genes related to fat metabolism.

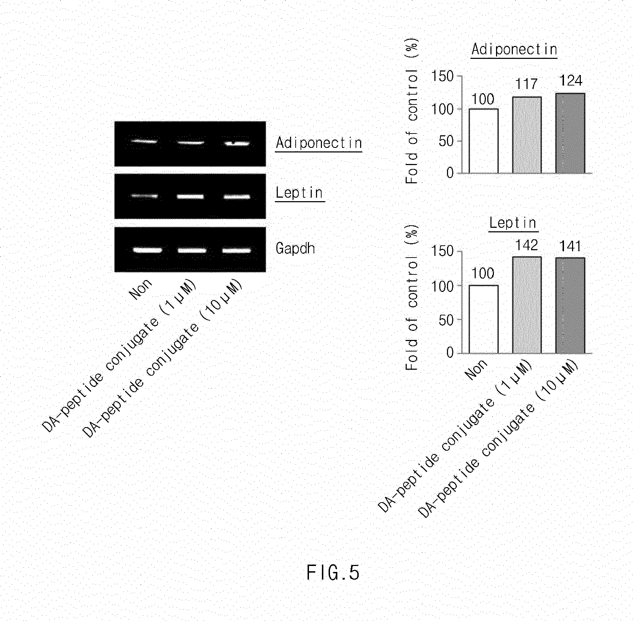

As a result, as illustrated in FIG. 5, it was confirmed that the expression of leptin and adiponectin genes, which were proteins related to fat metabolism, was increased by the treatment of the deoxycholic acid-peptide conjugate.

As a result, it can be seen that the deoxycholic acid-peptide conjugate of the present invention promotes the metabolism of fat necessary for energy generation, thereby inhibiting fat accumulation or promoting lipolysis.

### 4-3. Confirmation of increased expression of lipolysis-related genes using adipocytes

In adipocytes, in order to determine whether the deoxycholic acid-peptide conjugate had an activating effect on a cell signaling pathway for promoting lipolysis, the mRNA expression levels of genes related to promotion of fatty acid oxidation and promotion of lipolysis were confirmed.

Specifically, the differentiated adipocytes obtained in Preparation Example 1 were treated with 1 µM and 10 µM of the deoxycholic acid-peptide conjugate, and then cultured for 24 hours. After the cells were recovered and the polymerase chain reaction was performed by the method described in Experimental Example 4-1, the expression products were loaded on an agarose gel to confirm the mRNA expression levels of genes related to a lipolysis-promoting cell signaling pathway.

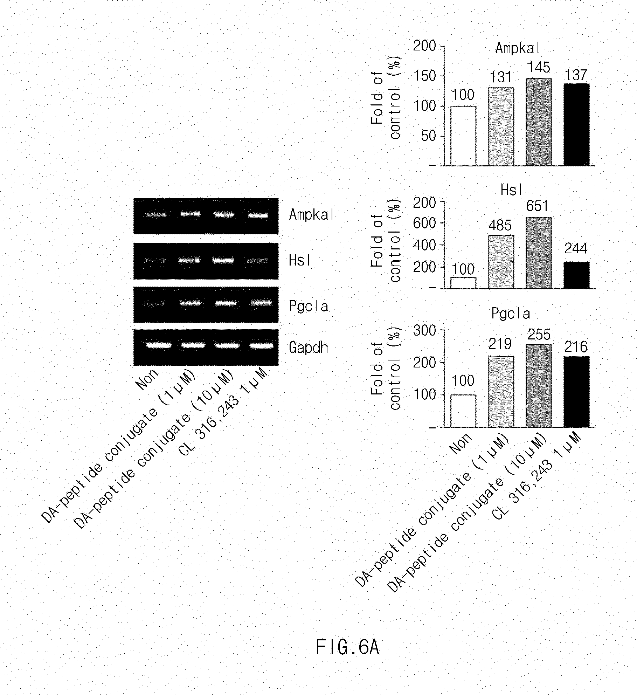

As a result, as illustrated in FIG. 6A, the expression of a lipolysis marker HSL was increased after treatment with the deoxycholic acid-peptide conjugate, and the expression of an upper signaling factor AMPKa1 and a lower factor thereof PGC1a related to fat metabolism was increased.

As a result, it can be seen that the deoxycholic acid-peptide conjugate according to the present invention increases the expression of genes related to lipolysis or oxidation promotion of fatty acids so as to very effectively decompose the fat.

### 4-4. Confirmation of increased expression of lipolysis genes using adipose tissue

In adipose tissue, in order to determine whether the deoxycholic acid-peptide conjugate had an activating effect on a cell signaling pathway for promoting lipolysis, the mRNA expression levels of genes related to promotion of fatty acid oxidation and promotion of lipolysis were confirmed.

Specifically, the adipose tissue obtained in Preparation Example 2 was treated with 1 µM and 10 µM of the deoxycholic acid-peptide conjugate and 1 µM of CL316243 (Sigma, USA) as a positive control group and cultured for 72 hours. After the cells were recovered and the polymerase chain reaction was performed by the method described in Experimental Example 4-1, the expression products were loaded on an agarose gel to confirm the mRNA expression levels of genes related to the promotion of fatty acid oxidation and the promotion of lipolysis.

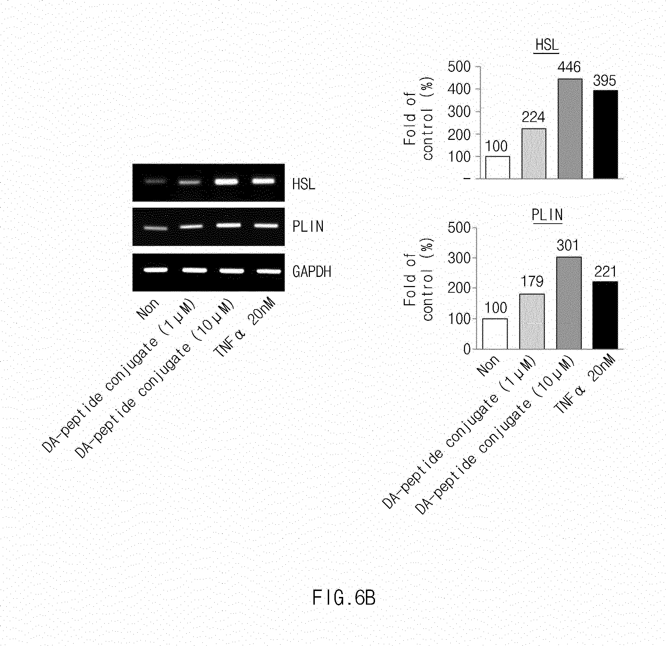

As a result, as illustrated in FIG. 6B, the expression of fat-related genes HSL and PLIN was increased in a concentration dependent manner after treatment with the deoxycholic acid-peptide conjugate.

As a result, it can be seen that the deoxycholic acid-peptide conjugate according to the present invention increases the expression of genes related to lipolysis or oxidation promotion of fatty acids so as to very effectively decompose the fat.

### 4-5. Confirmation of decreased expression of adipogenesis gene using adipose tissue

In adipose tissue, in order to confirm whether the deoxycholic acid-peptide conjugate had an effect of inhibiting adipogenesis, the mRNA expression levels of genes related to adipogenesis were confirmed.

Specifically, the adipose tissue obtained in Preparation Example 2 was treated with 1 µM and 10 µM of the deoxycholic acid-peptide conjugate and 1 µM of CL316,243 (Sigma, USA) as a positive control group and further cultured for 72 hours. After the cells were recovered and the polymerase chain reaction was performed by the method described in Experimental Example 4-1, the expression products were loaded on an agarose gel to confirm the mRNA expression levels of genes related to adipogenesis.

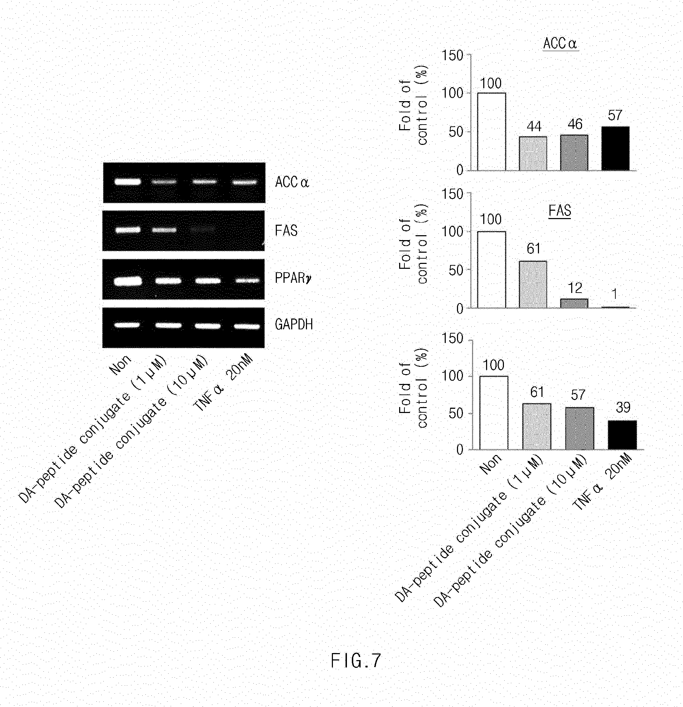

As a result, as illustrated in FIG. 7, it was confirmed that the expression of adipogenesis-related genes ACCα, FAS, and PPARγ was decreased after treatment with the deoxycholic acid-peptide conjugate.

As a result, it can be seen that the deoxycholic acid-peptide conjugate according to the present invention has an effect of preventing or treating obesity by inhibiting adipogenesis.

### [Experimental Example 5]

### Confirmation of increased expression of thermogenesis-related genes by deoxycholic acid-peptide conjugate in muscle cells

In order to confirm whether the deoxycholic acid-peptide conjugate of the present invention had an effect of preventing or treating obesity, the mRNA expression levels of thermogenesis-related genes of brown adipocytes were confirmed.

Specifically, the adipose tissue obtained in Preparation Example 2 was treated with 1 µM and 10 µM of the deoxycholic acid-peptide conjugate and 1 µM of CL316,243 (Sigma, USA) as a positive control group and further cultured for 72 hours. After the cells were recovered and the polymerase chain reaction was performed by the method described in Experimental Example 4-1, the expression products were loaded on an agarose gel to confirm the mRNA expression levels of genes related to adipogenesis. At this time, a GAPDH gene was used as an internal control group, and primers used in the experiment were shown in Table 2 below.

**[Table 2]**

| Gene | Primer | Sequence (5' → 3') | SEQ ID NO: |
|---|---|---|---|
| UCP1 | F | CCTGCCTCTCTCGGAAACAA | 24 |
| | R | GTAGCGGGGTTT ATCCCAT | 25 |
| PRDM | F | CCCCACATTCCGCTGTGAT | 26 |
| | R | CTCGCAATCCTTGCACTCA | 27 |
| Cidea | F | CGGGAATAGCCAGAGTCACC | 28 |
| | R | TGTGCATCGGATGTCGTAGG | 29 |

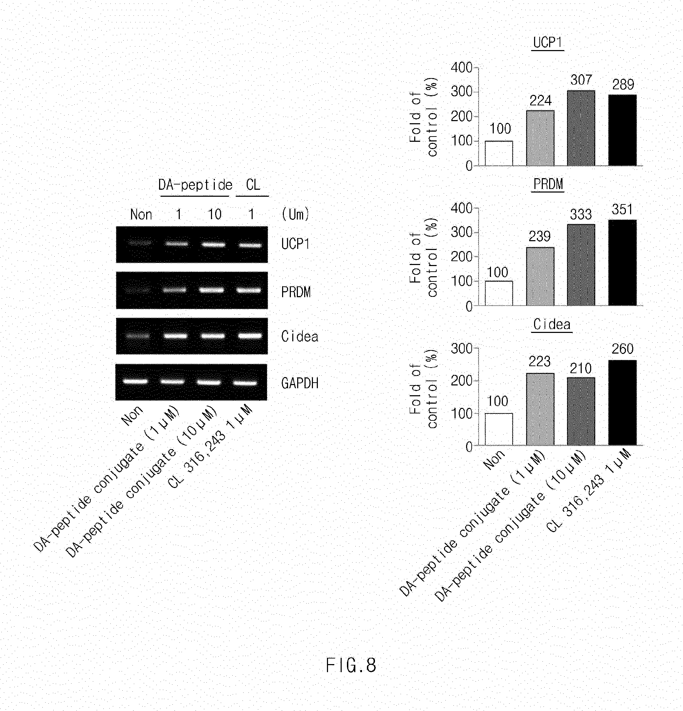

As a result, as illustrated in FIG. 8, it was confirmed that the expression of thermogenesis-related genes UCP1, PRDM, and Cidea of the brown adipocytes was increased after treatment with the deoxycholic acid-peptide conjugate. As a result, it can be seen that the deoxycholic acid-peptide conjugate according to the present invention increases the expression of the thermogenesis-related genes of the brown adipocytes whose expression is increased by exercise to have an effect of preventing or treating obesity.

### [Experimental Example 6]

### Confirmation of increased expression of IL-15 gene by deoxycholic acid-peptide conjugate in muscle cells

In order to confirm whether the deoxycholic acid-peptide conjugate of the present invention had an effect of preventing or treating obesity, expression changes in IL-15 gene in muscle cells were confirmed. IL-15 is a myokine whose expression level is increased by muscle contraction during resistive exercise and known to play a key role in the interaction between muscle and adipose tissue, and has an anti-obesity effect by inhibiting lipid accumulation.

Specifically, C2C12 cells, which were mouse myoblasts, were inoculated into a 6-well plate at a density of 2 × 10⁵ cells/well, and then cultured in a DMEM (10% FBS) medium for 3 days. When the cells had grown to about 70 to 80% of the plate, the medium was replaced with a differentiation medium (DMEM, 5% horse serum) and then cultured for 3 days. Thereafter, the medium was replaced with the differentiation medium (DMEM, 2% horse serum) and cultured for 4 days to induce differentiation from myoblasts to myotubes. Then, the medium was replaced with a serum-free medium and cultured for 2 hours, and treated with 1 µM and 10 µM of the deoxycholic acid-peptide conjugate for 6 hours and cultured in an environment of 37°C and 5% CO₂. After the cells were recovered and the polymerase chain reaction was performed by the method described in Experimental Example 4-1, the expression products were loaded on an agarose gel to confirm the mRNA expression levels of genes related to adipogenesis. At this time, a GAPDH gene was used as an internal control group, and primers used in the experiment were shown in Table 3 below.

**[Table 3]**

| Gene | Primer | Sequence (5' → 3') | SEQ ID NO: |
|---|---|---|---|
| IL-15 | F | TGTCTTCATTTTGGGCTGTG | 30 |
| | R | TGCAACTGGGATGAAAGTCA | 31 |

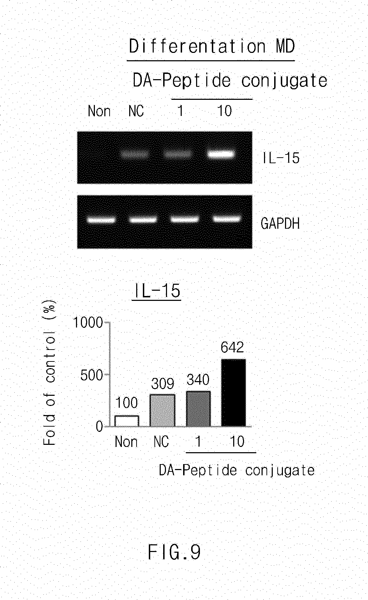

As a result, as illustrated in FIG. 9, it was confirmed that the expression of IL-15 having a lipolysis function of adipose tissue was increased after treatment with the deoxycholic acid-peptide conjugate.

As a result, it can be seen that the deoxycholic acid-peptide conjugate according to the present invention increases the expression of the IL-15 gene expressed by exercise and having the lipolysis function to have an effect of preventing or treating obesity.

As described above, while the present invention has been described in detail with reference to the described embodiments, it is apparent to those skilled in the art that various modifications and changes can be made within the technical idea of the present invention, and it is natural that these modifications and changes belong to the appended claims.

## Claims

1. A deoxycholic acid-peptide conjugate in which deoxycholic acid is conjugated with a peptide consisting of an amino acid sequence of SEQ ID NO: 1.

2. The deoxycholic acid-peptide conjugate of claim 1, wherein the deoxycholic acid is peptide-bound with an N-terminus of the peptide.

3. The deoxycholic acid-peptide conjugate of claim 1, wherein the deoxycholic acid-peptide conjugate inhibits adipogenesis in adipocytes.

4. The deoxycholic acid-peptide conjugate of claim 1, wherein the deoxycholic acid-peptide conjugate promotes lipolysis.

5. The deoxycholic acid-peptide conjugate of claim 1, wherein the deoxycholic acid-peptide conjugate reduces the sizes of adipocytes.

6. A composition for inhibiting adipogenesis or promoting lipolysis comprising the deoxycholic acid-peptide conjugate of claim 1.

7. A composition for preventing, treating or ameliorating obesity or obesity-related diseases comprising the deoxycholic acid-peptide conjugate of claim 1.

8. A pharmaceutical composition for preventing or treating obesity comprising the deoxycholic acid-peptide conjugate of claim 1.

9. The pharmaceutical composition of claim 8, further comprising:
a pharmaceutically acceptable carrier, an excipient, or a diluent.

10. The pharmaceutical composition of claim 8, wherein the carrier is at least one selected from the group consisting of saline, sterile water, a Ringer's solution, buffered saline, a dextrose solution, a maltodextrin solution, glycerol, and ethanol.

11. The pharmaceutical composition of claim 8, wherein the pharmaceutical composition is formulated in the form of oral preparations, injectable preparations or external preparations.

12. The pharmaceutical composition of claim 11, wherein the injectable preparation is for removing localized fat deposition.

13. The pharmaceutical composition of claim 12, wherein the localized fat is localized in a target area selected from the group consisting of the abdomen, under the eyes, under the chin, under the arms, buttocks, thighs, calves, back, thighs, and bra line.

14. A cosmetic composition for preventing or ameliorating obesity comprising the deoxycholic acid-peptide conjugate of claim 1.

15. The cosmetic composition of claim 14, wherein the cosmetic composition has at least one formulation selected from the group consisting of a skin, a lotion, a cream, an essence, an emulsion, a gel, a hand cream, a lipstick, a cleansing foam, a cleansing cream, cleansing water, a spray, a shampoo, a rinse, a treatment, a body cleanser, a soap, a pack, a massage agent, a face powder, a compact, a foundation, a two-way cake, and a makeup base.
